# EUROPEAN PATENT SPECIFICATION

(11) **EP 1 341 726 B1**
(45) Date of publication and mention of the grant of the patent: **22.02.2006**
(21) Application number: 01270501.8
(22) Date of filing: 13.12.2001
(51) Int. Cl.: C02F 1/48, C02F 1/46

(54) **DECONTAMINATED FLUIDS AND BIOCIDAL LIQUIDS**
DEKONTAMINIERTE FLUIDE UND BIOZIDE FLÜSSIGKEITEN
FLUIDES DESINFECTES ET LIQUIDES BIOCIDES

(30) Priority: 16.12.2000 GB 0030740
(43) Date of publication of application: 10.09.2003
(73) Proprietor: University of Strathclyde, Glasgow G1 3AE (GB)
(72) Inventor: MACGREGOR, Scott John, Glasgow G72 8NF (GB)
(74) Representative: MacDougall, Donald Carmichael
(86) International application number: PCT/GB2001/005535
(87) International publication number: WO 2002/048053

(56) References cited:
- EP-A- 0 431 190
- WO-A-99/47230
- DE-A- 10 030 735
- GB-A- 2 328 133
- US-A- 4 384 943
- US-A- 5 630 915
- US-A- 5 635 059
- US-A- 5 766 447

## Description

The present invention relates to a method for the purification of contaminated gases and liquids and for the production of biocidal liquids that have a finite activity period.

As is well known there are many applications for purified or decontaminated liquids. Water, for example, can be purified or decontaminated to potable standards and is then fit for human consumption. Alternatively water can be disinfected to remove a variety of pathogens such as those which cause Legionnaires' disease. It is also well known there are many applications for biocidal liquids which can be used to inactivate microorganisms on contact and food surfaces, in liquids and the like, where a treated liquid may be poured on to surfaces or into liquids to inactivate microorganisms.

At the present time the principal purification or decontamination methods involve chemical and/or heat treatment. In the case of water, chlorination (that is dosing the contaminated water with chlorine) is widely used but the process leaves residues which have to be filtered out and also unpleasant tastes which are difficult to eliminate. Additionally, the inevitable escape of chlorine to the atmosphere ,is environmentally hazardous. An alternative treatment process involves UV irradiation, but this is limited to a relatively thin film liquid flow of no more than a few centimetres and is, therefore, less suitable for bulk liquid treatment. A further alternative purification or decontamination process involves dosing with ozone, but this is complex because ozone is a highly unstable gas and cannot be stored. The ozone has to be produced in a specially designed generator and then immediately dissolved in the liquid to be treated. A still further option is to introduce bubbles of gas within the liquid and apply a pulsed electrical field to cause ionisation activity. Arrangements of this general type are described in WO 99/47230, GB 2,328,133, US 5,630,915 and US 5,766,477.

It is an object of the present invention to provide for the production of a purified or decontaminated fluid in a single simple aeration or sparging process wherein ozone and Uv radiation are generated and act synergistically within a liquid.

It is a further object of an aspect of this invention to remove the necessity of barrier filters in air cleaning devices, which act as collecting and breeding grounds for air borne pathogens.

It is an object of a further aspect of this invention to produce a biocidal liquid with a finite activity duration that becomes non-biocidal with time.

The present invention provides a method for producing a purified or decontaminated fluid or a biocidal liquid with a finite activity duration, as defined in claim 1.

The resulting liquid may be utilised as a biocidal liquid, or in other applications requiring purified, decontaminated, or sterilised liquid. Alternatively, the method may be utilised to treat gases that contain microbial particles and cell matter, for filtering air for high cleanliness areas, or to remove biological material from air supplies such as in hospitals and military installations, such gases being sparged into the liquid of the method.

By virtue of the present invention ozone and UV radiation are generated directly within the body of fluid under treatment without the need for a separate ozone generator and subsequent mixer to dissolve the ozone in the fluid. The fluid is treated using a single process which combines the functions of ozone generation and ozone dissolution. For decontamination purposes the process exploits the synergism of the two sterilising agents ozone and UV irradiation in removing pathogens from the fluid. If the pulses are sufficiently long electroporosis of remnant pathogens in the liquid additionally occurs.

The electrical field may take the form of unidirectional pulses of short duration, short pulses of alternating polarity or a conventional sinusoid.

The aerating or sparging gas may be air and preferably is ionised negatively before injection into the liquid. Preferably the gas will be carbon dioxide (CO₂), Nitrogen (N₂), Oxygen (O₂) or Air. CO₂ is useful as it is mildly acidic and biocidal, whereas N₂ is useful because it produces a very high UV content, whilst Oxygen is desirable for producing ozone, and Air is useful because it is cheap and contains Oxygen to produce ozone. The bubble diameter will be sufficiently large as to permit ionisation activity in the gas within it. That is to say the diameter will be greater than that corresponding to the Paschen minimum for gas discharge in the particular gas. Typically for oxygen the bubble diameter will be greater than 10µm but will for convenience preferably lie in the range 100µm to 3000µm. The ionisation process produces a release of monoatomic oxygen plus free radicals and other gaseous species and the ratio of gas-to-liquid flow preferably lies in the range 0.05 to 0.2 so that for small bubbles of 100*µ*m diameter the bubble density would lie in the range 100,000 per cc of liquid to 400,000 per cc of liquid. Such densities are difficult to achieve. However for bubbles of 500µm diameter the densities lie in the range 800 per cc of liquid to 3000 per cc of liquid and such densities are quite readily achieved. For large bubble diameters the corresponding density figures are:
1000*µ*m diameter : 100 to 400 per cc of liquid
2000*µ*m diameter : 12 to 48 per cc of liquid
3000*µ*m diameter : 4 to 14 per cc of liquid.

The pulses of electric field of unipolarity or alternating polarity will each have a duration sufficiently long to create ionisation activity within the gas bubbles with the associated generation of ozone but preferably not long enough to allow significant conduction current to flow in the liquid. Typically for water, the pulse duration will preferably lie in the range of a few nanoseconds (say 1 to 10ns) up to about 500ns but this range will be different for different liquids. For the case of conventional sinusoidally alternating field, the frequency of the sinusoid will be governed by the same criteria as for the pulsed field. Typically for water the frequency will be preferably in the range 2.5MHz to 250 MHz.

The delivered dose of ozone to the fluid to be treated is preferably in the range 2 to 24 mg per litre, although for most purposes a dose in the range 4 to 10 mg per litre will be sufficient. Such doses can be achieved by varying the frequency of pulsing the electric field and/or by recirculating and re-aerating the fluid under treatment, the optimal dose being a matter of trial and experiment for any particular bubble diameter and bubble density depending upon the size of the exposure chamber and the applied voltage.

The half-life of dissolved Ozone is strongly dependent upon temperature but is typically around 30-60 minutes at room temperature. It is envisaged that the biocidal wash will be applied at room temperature and under such conditions, in the absence of contact with any oxidising material, the biocidal wash will remain active for approximately 2-3 times the half-life, namely 1-2 hours. Of course, the biocidal wash may be applied at other temperatures, typically within the range 10 to 50°C, depending upon the requirements of the item to be washed.

Typically with oxygen as the aerating gas, an electric field preferably in excess of 25kV/cm will be applied to the gas.

The present invention also provides apparatus for producing purified or decontaminated fluid or a biocidal liquid as defined in claim 9.

The means for pre-ionising the flow of gas to the mixer device are conveniently to produce a negatively ionised gas.

It will be seen that the invention embodies a system which is robust, employs no fragile dielectric barriers and thus obviates all the attendant mechanical failure problems and maintenance requirements associated with dielectric barriers.

The apparatus may further comprise means for enabling the liquid under treatment to be pressurised up to a range in the order of 10-15 atmospheres, although it would be normal to operate such apparatus at about 1 atmosphere. The increase in liquid pressure facilitates an increase in the amount of gas capture possible within the body of the liquid, thus increasing the biocidal potential of the liquid.

It may also be desirable to vary the pressure on the body of liquid over a period of time which may be cyclic in nature, that is to say to pressurise and then de-pressurise the liquid. Likewise it may be desirable to vary the intensity of the electric field on the body of liquid during any part of the aforementioned pressure cycle.

Embodiments of the present invention will now be described by way of example with reference to the accompanying drawings in which:
Fig 1 illustrates apparatus in accordance with the present invention for producing purified or decontaminated fluid or a biocidal liquid;
Fig 2 illustrates a modified form of the Fig 1 apparatus incorporating re-circulation of the liquid under treatment;
Fig 3 illustrates operation of a detail of the Figs 1 and 2 apparatus; and
Fig 4 illustrates three different configurations for the exposure chamber used in Figs 1 and 2.

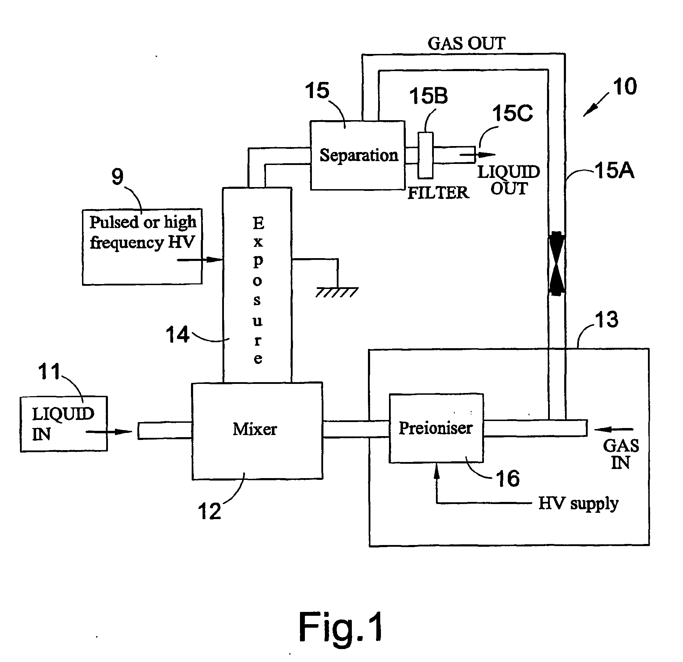

As is shown in Fig 1 apparatus 10 for producing purified or decontaminated liquid or a biocidal liquid comprises a pump 11 for delivering a flow of liquid to be acted upon to a mixer device 12 which also receives a flow of gas from a source 13. The mixer 12 aerates or sparges the liquid with bubbles of gas which are preferably distributed throughout the liquid, and delivers it to an exposure chamber 14 where the aerated or sparged liquid is subjected to either a sequence of very short high-voltage electrical pulses of unipolarity or alternating polarity or a sinusoidal ac high frequency voltage delivered by a source 9. One suitable form of mixer 12 is a statiflow mixer which provides controllable bubble size and density.

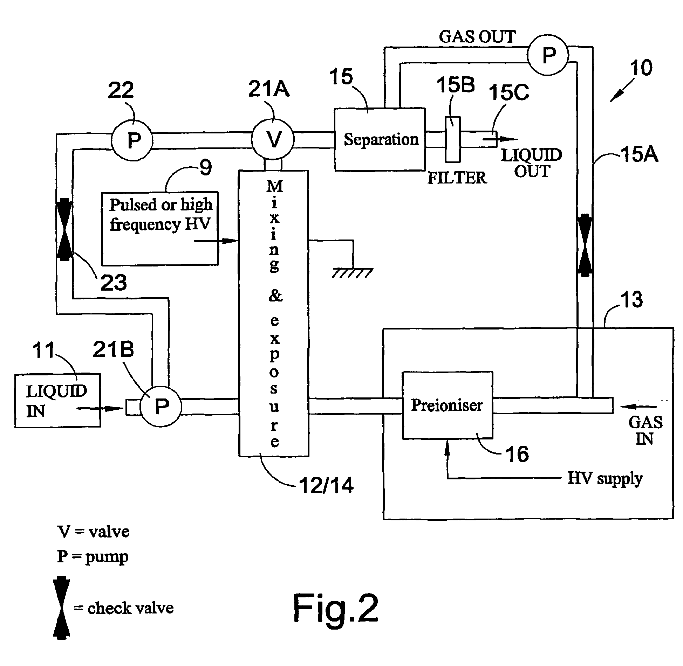

Alternatively the mixer 12 and exposure chamber 14 may be combined as in Fig 2 (12/14) such that the exposure to the high voltage application takes place within the mixing device.

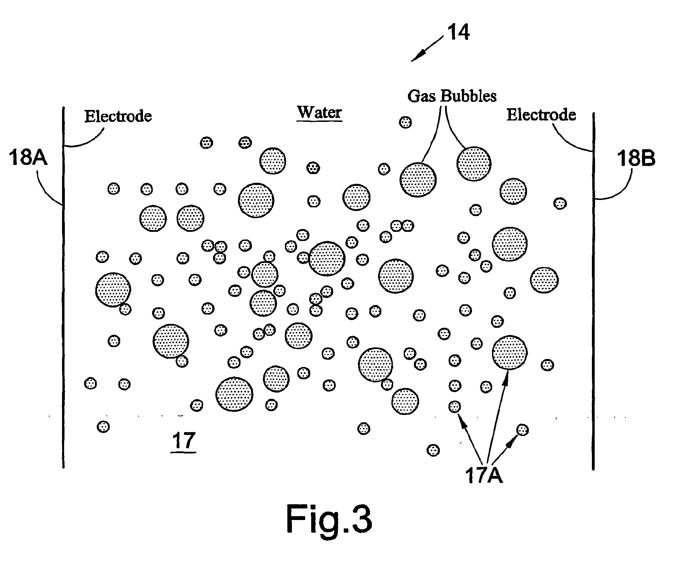

The interior of chamber 14 is schematically shown in Fig 3 from which it will be seen that the chamber 14 is provided with electrodes 18A, 18B between which the aerated liquid 17 is caused to flow. The aeration is such as to produce a widely dispersed suspension of gas bubbles 17A greater than 10µm in diameter and most preferably in the range 100µm to 1000µm in diameter. When high voltage electrical pulses of very short duration are applied across the electrodes 18A, 18B (and provided the pulse duration is sufficiently short) the liquid acts uniquely as a dielectric having high permitivity so that a relatively high electrical field is applied to the gas within the bubbles 17A. Electrical discharges and breakdown, therefore, take place in the gas bubbles 17A with the associated generation of ozone and emission of UV irradiation. After breakdown of the gas within the bubbles 17A the high voltage of the supply is effectively impressed across the liquid functioning as a relatively non-conductive dielectric to thereby produce electroporosis of remnant pathogens in the liquid if the pulse is sufficiently long. Typically for water, since the permitivity of water is a factor of 80 greater than the permitivity of gas, the electric field across the gas bubble 17A is about 80 times the field in the liquid 17 prior to ionisation of the bubbles 17A. The production of ozone and UV within the bubbles 17A results in purification of the associated fluid and because it takes place in a distributed fashion throughout the body of the liquid 17 highly efficient purification results.

With further reference to Fig 1 the treated fluid emerging from the exposure chamber 14 is preferably delivered to a separator unit 15 where at least the majority of the remnant aeration is extracted and pumped via passageway 15A (which incorporates a check-valve) to the input gas source 13 for recycling. The purified biocidal liquid is then delivered from the unit 15 through a filter 15B (which may embody a catalytic destructor) to output 15C.

The input gas source 13 incorporates a pre-ioniser 16 preferably operated with a negative HV electrical supply to cause the gas within the bubbles 17A initially to be negatively charged, which leads to a rapid ionisation of the gas within the bubbles when the high frequency or short duration pulsed voltage is applied to the chamber 14, the ionisation process producing a release of monoatomic oxygen plus free radicals and other gaseous species within the liquid.

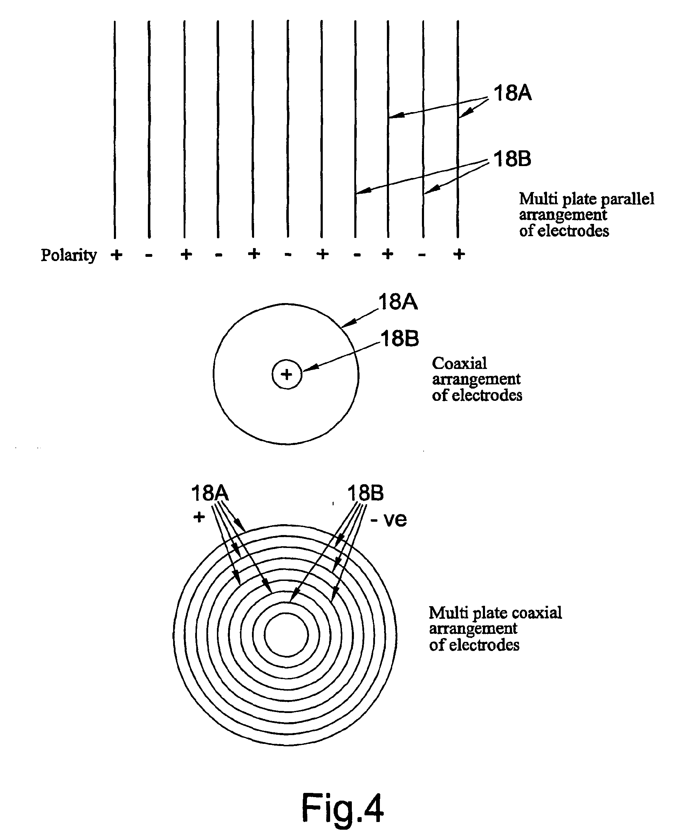

The electrodes 18A, 18B in the chamber 14 may be of rectangular or concentric geometry and may also take the form of multiple-plates of alternating positive and negative polarity three different configurations of which are shown in Fig 4. The electrodes, regardless of their geometry, may either be in contact with the liquid to be treated or isolated from it by a layer of dielectric material preferably having a dielectric permitivity which is high in comparison with the dielectric permitivity of the liquid to be treated, and preferably having a conductivity which is low in comparison with the conductivity of the liquid to be treated. A dielectric material such as barium titanate is preferable. Ceramic dielectric materials may also be used particularly where the liquid under treatment might attack and damage the metal electrodes.

The voltage applied to the electrodes is such as to create an electrical field in the gas within the bubbles of preferably greater than 25kV/cm. The electrical pulses must have a duration sufficiently long to create ionisation within the bubbles but not necessarily long enough to cause significant conduction current in the liquid. Typically for water the pulses have a duration in the range from a few nanoseconds up to 500ns for which the water functions principally as a dielectric. If the voltage pulse has a duration significantly greater than 500ns then the water acts as partly dielectric partly conductive and, accordingly, an unacceptably high conductive current flows in the liquid to be treated. Such a conductive current results in unwanted energy dissipation in the liquid to be treated and, as such, represents a reduced efficiency. Where the liquid is water pulse durations around 50, 60, 70, 80, 90 or 100ns are preferred.

Fig 2 illustrates a modified form of the Fig 1 apparatus wherein common components have like numerals. In Fig 2 the mixer and exposure chamber 12/14 are combined as previously mentioned but additionally provision is made for the re-circulation of liquid under treatment. This is achieved by passageway 20 connected at either end via valves 21A, 21B and containing a pump 22 and a check valve 23. Valve 21A located at the output of the unit 12/14 is operable to direct the output of unit 12/14 to the separator 15 as in Fig 1 or to the passageway 20 for re-circulation. Valve 21B located at the liquid inlet of unit 12/14 is operable to deliver liquid to unit 12/14 as in Fig 1 or from passageway 20. Valves 21A, 21B may be ganged and preferably are two-position valves.

The biocidal liquid produced has a finite useable activity duration which can be determined from the half-life of dissolved Ozone. One factor upon which the half-life is dependant upon is temperature, however, the half-life of the ozone is typically around 30-60 minutes at room temperature, at which temperature the biocidal liquid will remain active for approximately 2-3 times the half-life of the Ozone, namely 1-3 hours, if the liquid does not come into contact with any oxidising material.

Tables 1A and 1B show how a biocidal liquid becomes non-biocidal with time. In Table 1A the biocidal liquid was obtained using CO₂ gas to aerate 500ml solution of Peptone dissolved in water, using the process described herein above for a duration of 50 seconds. *Bacillus cereus* KD1 was then added to samples of the biocidal liquid, at the times shown after treatment. Population samples were taken and the cell count (CFU's) of each sample after incubation are recorded in Table 1A.

These results show how the treated Peptone solution becomes non-biocidal with time, as during the first two hours the solution can eliminate 99% of the *Bacillus cereus* KD1, whereas after 3 hours the solution eliminates less than 10% of the *Bacillus cereus* KD1.

In Table 1B the biocidal liquid was obtained using oxygen and water. The bacterium *Campylobacter jejuni* was added at different times after biocidal liquid generation and the resulting bacterial inactivation measured as before.

The biocidal liquid may be applied to surface or articles, which are to be treated in a number of ways. The biocidal liquid may be poured onto the surfaces, articles may be dipped in the biocidal liquid, or the biocidal liquid may be used in a wash-down process used to clean apparatus. The biocidal liquid may be applied at temperatures other than room temperature in order to benefit from synergistic thermal effects or enhanced ozone solubility.

Once the biocidal liquid is applied it will immediately, upon contact with any oxidisable material, react thereby inactivating microorganisms. Table 2 provides results showing the effective reduction of certain organisms with biocidal liquid aerated with different gases.

It will be appreciated from the foregoing that if the liquid is immune to the effects of ozone then the remnant aeration which is extracted from the separator unit 15 will be ozone-rich and the system accordingly functions as an ozone generator (without use of a dielectric barrier). The present invention is therefore also directed to such a system functioning as an ozone generator, the gaseous output from which may be used in any conventional manner.

Furthermore this method can be utilised for the treatment of contaminated gases, where a contaminated gas is used to aerate a liquid medium and the ionisation activity within the gas bubbles eliminates any biological activity within the gas. Such a method may be utilised to clean or scrub dirty gases that may be produced as a bi-product of a process or production plant, such as incinerators and the like, where the gases may contain toxic or polluting agents. These toxic/pollutant components can be neutralised from the gas stream by this method, thus allowing the release of a cleaned or scrubbed air stream into the atmosphere.

**Table 1A**

| **Time after Treatment** | **Cell Count (CFU/ml)** | **% Reduction** |
|---|---|---|
| Untreated | 9.2 x 10⁵ | N/A |
| ~ 5 mins | 7.0 X 10¹ | 99.99 |
| 1 hr | 1.2 x 10³ | 99.98 |
| 2 hr | 1.1 x 10⁴ | 98.8 |
| 3 hr | 3.2 x 10⁵ | 7 |
| 4 hr | 8.6 x 10⁵ | 6.5 |

**Table 1B**

| **Time after Treatment** | **Cell Count (CFU/ml)** | **% Reduction** |
|---|---|---|
| Untreated | 5.9 x 10⁸ | N/A |
| 1 min | 6.1 x 10¹ | > 99.9999 |
| 10 mins | 1.6 x 10² | > 99.9999 |
| 25 mins | 3.1 x 10³ | > 99.999 |
| 60 mins | 1.2 x 10⁵ | > 99.9 |

**Table 2 Biocidal Activity In Treated Liquids**

| **ORGANISM** | **LIQUID** | **GAS** | **UNTREATED** | **TREATED** | **% RED'N** |
|---|---|---|---|---|---|
| *Bacillus cereus KD1* | 1 | N₂ | 1.0 × 10⁶ | 8.6 × 10³ | 99.14 |
| | | | | | |
| *Bacillus cereus KD1* | 1 | CO₂ | 1.2 X 10⁵ | 1.1 × 10³ | 99.08 |
| | | | | | |
| *Bacillus cereus KD1* | 1 | AIR | 2.6 X 10⁴ | 1.1 X 10² | 99.58 |
| | | | | | |
| *Escherichia coli* | 2 | N₂ | 3.5 × 10⁶ | 7.5 × 10³ | >99.7 |
| | | | | | |
| *Escherichia coli* | 2 | CO₂ | 1.5 × 10⁶ | 3.1 x 10⁴ | >97.9 |
| | | | | | |
| *Escherichia coli* | 2 | AIR | 1.6 x 10⁶ | 1.5 × 10³ | >99.9 |
| | | | | | |
| *Campylobacter jejuni* | 2 | O₂ | 5.3 x 10⁸ | 6.2 x 10¹ | >99.9999 |

| | | | | | |
|---|---|---|---|---|---|
| where liquid 1 is a solution of Peptone in water, and liquid 2 is distilled water. | | | | | |

## Claims

1. A method for producing a purified or decontaminated fluid or a biocidal liquid with a finite activity duration, the method comprising aerating or sparging a liquid (17) with a gas, the sparging being such as to provide a suspension of gaseous bubbles (17A) within the liquid, and subjecting the sparged liquid to a pulsed electrical field to create ionisation activity in the gas bubbles (17A), the method being **characterised by** ionising the gas prior to aerating or sparging the liquid (17).

2. A method as claimed in claim 1 wherein the gas is negatively ionised prior to aerating or sparging the liquid.

3. A method as claimed in claim 1 or claim 2 wherein the pulse duration of the electrical pulses is in the range 1 to 500ns.

4. A method as claimed in any of the preceding claims, wherein the electrical field is in the form of unidirectional pulses of short duration, short pulses of alternating polarity, or a conventional sinusoid.

5. A method as claimed in any of the preceding claims, wherein the aerating or sparging gas is carbon dioxide (CO₂), Oxygen (O₂) or Air.

6. A method as claimed in any one of the preceding claims, wherein the ratio of gas-to-liquid flow rate through the electrical field lies in the range 0.05 to 0.2.

7. A method as claimed in any preceding claim further comprising pressurising the sparged liquid.

8. A method as claimed in claim 7, wherein the pressurisation is cylical and the electrical field is varied during the pressurisation cycle.

9. An apparatus for producing a purified or decontaminated fluid or a biocidal liquid with a finite activity duration comprising means (11) for delivering a flow of liquid to a mixer device (12), means of delivering a flow of gas the mixer device (12), the mixer device (12) being arranged to aerate or sparge the liquid with the gas such as to provide a suspension of gas bubbles within the liquid, an exposure chamber (14) for receiving the aerated or sparged liquid (17) and arranged to subject the liquid to a pulsed electric field for creating ionisation activity in the gas bubbles, **characterised by** means for ionising the gas (16) prior to introducing it into the mixer device.

10. An apparatus as claimed in claim 9 wherein the pulse duration of the electrical field is in the range of 1ns to 500ns.

11. An apparatus as claimed in claim 9 or claim 10 comprising means for varying the frequency of pulsing the electric field.

12. An apparatus as claimed in any of claims 9 to 11 comprising means for re-circulating the treated liquid.

13. An apparatus as claimed in any of claims 9 to 12, further comprising pressurising means for pressurising the liquid under treatment.

14. An apparatus as claimed in claim 13, wherein the pressurising means are operable to vary the pressure cyclically.

15. An apparatus as claimed in claim 14 comprising means for varying the electric field during the pressure cycle.

## Patentansprüche

1. Verfahren zum Herstellen eines gereinigten oder dekontaminierten Fluids oder einer bioziden Flüssigkeit mit einer finiten Aktivitätsdauer, wobei das Verfahren Belüften oder Durchperlen einer Flüssigkeit (17) mit einem Gas umfasst, wobei durch das Durchperlen eine Suspension gasförmiger Blasen (17A) innerhalb der Flüssigkeit bereitgestellt wird, sowie Einbringen der durchperlten Flüssigkeit in ein gepulstes elektrisches Feld umfasst, um in den Gasblasen (17A) lonisierungsaktivität zu erzeugen, wobei das Verfahren **gekennzeichnet ist durch** Ionisieren des Gases vor Belüften oder Durchperlen der Flüssigkeit (17).

2. Verfahren nach Anspruch 1, wobei das Gas vor dem Belüften oder Durchperlen der Flüssigkeit negativ ionisiert wird.

3. verfahren nach Anspruch 1 oder Anspruch 2, wobei die pulsdauer der elektrischen Pulse im Bereich zwischen 1 bis 500 ns liegt.

4. verfahren nach einem der vorangehenden Ansprüche, wobei das elektrische Feld die Form gleichgerichteter Pulse von kurzer Dauer, kurzer Pulse alternierender Polarität oder einer herkömmlichen Sinuskurve hat.

5. verfahren nach einem der vorangehenden Ansprüche, wobei das belüftende oder durchperlende Gas Kohlendioxid (CO₂), sauerstoff (O₂) oder Luft ist.

6. Verfahren nach einem der vorangehenden Ansprüche, wobei das Verhältnis von Gas- zu Flüssigkeits-Flussrate durch das elektrische Feld im Bereich von 0,05 zu 0,2 liegt.

7. verfahren nach einem der vorangehenden Ansprüche, ferner umfassend Unter-Druck-Setzen der durchperlten Flüssigkeit.

8. Verfahren nach Anspruch 7, wobei das Unter-Druck-Setzen zyklisch erfolgt und das elektrische Feld während des Unter-Druck-Setzens verändert wird.

9. Vorrichtung zum Herstellen eines gereinigten oder dekontaminierten Fluids oder einer bioziden Flüssigkeit mit einer finiten Aktivitätsdauer, umfassend Mittel (11) zum Bereitstellen eines Flusses von Flüssigkeit zu einer Mischeinrichtung (12), Mittel zum Bereitstellen eines Flusses von Gas zu der Mischeinrichtung (12), wobei die Mischeinrichtung (12) eingerichtet ist, die Flüssigkeit mit dem Gas derart zu belüften oder zu durchperlen, dass eine Suspension von Gasblasen innerhalb der Flüssigkeit gebildet wird, eine Expositionskammer (14) zum Aufnehmen der Flüssigkeit (17), die belüftet oder durchperlt wurde, und wobei die Expositionskammer derart eingerichtet ist, die Flüssigkeit einem gepulsten elektrischen Feld auszusetzen, um Ionisierungsaktivität in den Gasblasen zu erzeugen, **gekennzeichnet durch** Mittel zum Ionisieren des Gases (16) vor Einbringen des Gases in die Mischeinrichtung.

10. Vorrichtung nach Anspruch 9, wobei die Pulsdauer der elektrischen Pulse im Bereich zwischen 1 ns bis 500 ns liegt.

11. Vorrichtung nach Anspruch 9 oder Anspruch 10, umfassend Mittel zum verändern der Pulsfrequenz des elektrischen Feldes.

12. Vorrichtung nach einem der Ansprüche 9 bis 11, umfassend Mittel zum erneuten Zirkulieren der behandelten Flüssigkeit.

13. Vorrichtung nach einem der Ansprüche 9 bis 12, ferner umfassend Druckbeaufschlagungsmittel, um die in Behandlung befindliche Flüssigkeit unter Druck zu setzen.

14. Vorrichtung nach Anspruch 13, wobei die Druckbeaufschlagungsmittel betreibbar sind, den Druck zyklisch zu verändern.

15. Vorrichtung nach Anspruch 14, umfassend Mittel zum Verändern des elektrischen Feldes während des Druckzyklus.

## Revendications

1. Procédé de production d'un fluide purifié ou désinfecté ou d'un liquide biocide avec une durée d'activité limitée, le procédé comprenant l'aération d'un liquide (17) avec un gaz ou le barbotage d'un gaz dans un liquide (17), le barbotage étant tel qu'il fournit une suspension de bulles de gaz (17A) à l'intérieur du liquide, et la soumission du liquide ayant subi le barbotage à un champ électrique pulsé pour créer une activité d'ionisation dans les bulles de gaz (17A), le procédé étant **caractérisé par** l'ionisation du gaz avant l'aération du liquide (17) ou le barbotage dans le liquide (17).

2. Procédé selon la revendication 1, dans lequel le gaz est ionisé négativement avant l'aération du liquide ou le barbotage dans le liquide.

3. Procédé selon la revendication 1 ou la revendication 2, dans lequel la durée de pulsation des pulsations électriques se trouve dans la plage de 1 à 500 ns.

4. Procédé selon l'une quelconque des revendications précédentes, dans lequel le champ électrique est sous la forme de pulsations unidirectionnelles de courte durée, de pulsations courtes de polarité alternée, ou d'une sinusoïde conventionnelle.

5. Procédé selon l'une quelconque des revendications précédentes, dans lequel le gaz d'aération ou de barbotage est le dioxyde de carbone (CO₂), l'oxygène (O₂) ou l'air.

6. Procédé selon l'une quelconque des revendications précédentes, dans lequel le rapport du débit gaz/liquide dans le champ électrique se trouve dans la plage de 0,05 à 0,2.

7. Procédé selon l'une quelconque des revendications précédentes, comprenant en outre la pressurisation du liquide ayant subi le barbotage.

8. Procédé selon la revendication 7, dans lequel la pressurisation est cyclique et le champ électrique varie durant le cycle de pressurisation.

9. Appareil de production d'un fluide purifié ou désinfecté ou d'un liquide biocide avec une durée d'activité limitée comprenant des moyens (11) de délivrance d'un flux de liquide à un dispositif mélangeur (12), des moyens de délivrance d'un flux de gaz au dispositif mélangeur (12) ; le dispositif mélangeur (12) étant arrangé pour l'aération du liquide avec le gaz ou le barbotage du gaz dans le liquide de manière à fournir une suspension de bulles de gaz à l'intérieur du liquide, une chambre d'exposition (14) destinée à recevoir le liquide ayant subi une aération ou un barbotage (17) et arrangée pour soumettre le liquide à un champ électrique pulsé pour créer une activité d'ionisation dans les bulles de gaz, **caractérisé par** des moyens d'ionisation du gaz (16) avant son introduction dans le dispositif mélangeur.

10. Appareil selon la revendication 9, dans lequel la durée de pulsation du champ électrique se trouve dans la plage de 1 ns à 500 ns.

11. Appareil selon la revendication 9 ou la revendication 10, comprenant des moyens destinés à faire varier la fréquence de pulsation du champ électrique.

12. Appareil selon l'une quelconque des revendications 9 à 11, comprenant des moyens destinés à faire re-circuler le liquide traité.

13. Appareil selon l'une quelconque des revendications 9 à 12, comprenant en outre des moyens de pressurisation destinés à pressuriser le liquide sous traitement.

14. Appareil selon la revendication 13, dans lequel les moyens de pressurisation peuvent fonctionner pour faire varier la pression de manière cyclique.

15. Appareil selon la revendication 14, comprenant des moyens destinés à faire varier le champ électrique durant le cycle de pression.
